(19) Europäisches Patentamt / European Patent Office / Office européen des brevets

(11) **EP 4 306 502 A1**

(12) **EUROPEAN PATENT APPLICATION**
published in accordance with Art. 153(4) EPC

(43) Date of publication:
**17.01.2024 Bulletin 2024/03**

(21) Application number: **22767026.2**

(22) Date of filing: **04.03.2022**

(51) International Patent Classification (IPC):
**C07C 1/04** (2006.01)      **C07C 9/04** (2006.01)
**C25B 1/23** (2021.01)

(52) Cooperative Patent Classification (CPC):
**C25B 15/08; C07C 1/04; C07C 9/04; C25B 1/00;
C25B 1/02; C25B 1/23; C25B 3/25; C25B 9/19;
C25B 9/23; C25B 9/70; C25B 15/02**

(86) International application number:
**PCT/JP2022/009406**

(87) International publication number:
**WO 2022/191069 (15.09.2022 Gazette 2022/37)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **11.03.2021 JP 2021039697**

(71) Applicant: **NGK Insulators, Ltd.
Nagoya-shi, Aichi 467-8530 (JP)**

(72) Inventors:
• **KAN, Hirofumi
Nagoya-shi, Aichi 467-8530 (JP)**
• **TORII, Atsushi
Nagoya-shi, Aichi 467-8530 (JP)**

(74) Representative: **MERH-IP Matias Erny Reichl
Hoffmann
Patentanwälte PartG mbB
Paul-Heyse-Strasse 29
80336 München (DE)**

(54) **METHANE PRODUCTION SYSTEM AND METHANE PRODUCTION METHOD**

(57)    A methane production system (1) includes a co-electrolysis/reforming cell (32) and a control unit (60) that controls the operating temperature of the co-electrolysis/reforming cell (32). The co-electrolysis/reforming cell (32) operates in either a co-electrolysis mode in which $H_2$ and CO are produced at a first electrode (2) from $CO_2$ and $H_2O$, or a reforming mode in which $CH_4$ is produced at the first electrode (2) from the $H_2$ and CO produced in the co-electrolysis mode. The control unit (60) makes the operating temperature of the co-electrolysis/reforming cell (32) in the reforming mode lower than the operating temperature of the co-electrolysis/reforming cell (32) in the co-electrolysis mode.

FIG. 1

**Description**

TECHNICAL FIELD

The present invention relates to a methane production system and a methane production method.

BACKGROUND ART

Patent Literature 1 discloses a solid oxide electrolysis cell (abbreviated as "SOEC" hereinafter) provided with a hydrogen electrode at which $H_2O$ is electrolyzed, an electrolyte capable of transferring $O^{2-}$, and an oxygen electrode at which $O_2$ is produced from $O^{2-}$ transferred from the hydrogen electrode through the electrolyte.

Patent Literature 2 discloses that $H_2$ and CO can be produced by co-electrolyzing $CO_z$ and $H_2O$ at the hydrogen electrode of an SOEC.

CITATION LIST

Patent Literature

Patent Literature 1: JP 2018-154864A
Patent Literature 2: JP 2019-175636A

SUMMARY

TECHNICAL PROBLEM

In order to utilize, as fuel, $H_2$ and CO produced from $CO_z$ and $H_2O$ through co-electrolysis using an SOEC, it is effective to produce $CH_4$ from $H_2$ and CO using a reforming device.

However, in order to transport $H_2$ and CO from a plant where an SOEC is installed to a plant where a reforming device is installed, $H_2$ and CO need to be separated and liquefied.

Therefore, there is demand for performing production of $H_2$ and CO and production of $CH_4$ on-site (i.e., in one facility).

The present invention has been made in view of the above-described circumstances, and aims to provide a methane production system and a methane production method with which $H_2$, CO, and $CH_4$ can be produced on-site.

SOLUTION TO PROBLEM

A methane production system according to the present invention includes a co-electrolysis/reforming cell and a control unit that controls operating temperatures of the co-electrolysis/reforming cell. The co-electrolysis/reforming cell includes a first electrode, a second electrode, and an electrolyte disposed between the first electrode and the second electrode. The co-electrolysis/reforming cell operates in either a co-electrolysis mode in which $H_2$ and CO are produced at the first electrode from $CO_z$ and $H_zO$, or a reforming mode in which $CH_4$ is produced at the first electrode from the $H_2$ and CO produced in the co-electrolysis mode. The control unit makes an operating temperature of the co-electrolysis/reforming cell in the reforming mode lower than an operating temperature of the co-electrolysis/reforming cell in the co-electrolysis mode.

ADVANTAGEOUS EFFECTS

According to the present invention, it is possible to provide a methane production system and a methane production method with which $H_2$, CO, and $CH_4$ can be produced on-site.

BRIEF DESCRIPTION OF DRAWINGS

FIG. 1 is a block diagram showing a configuration of a methane production system.
FIG. 2 is a perspective view of a co-electrolysis/reforming device.

FIG. 3 is a cross-sectional view of the co-electrolysis/reforming device.
FIG. 4 is a perspective view of the co-electrolysis/reforming cell.
FIG. 5 is a cross-sectional view of the co-electrolysis/reforming cell.
FIG. 6 is a flowchart illustrating a methane production system.

DESCRIPTION OF EMBODIMENTS

Methane production system

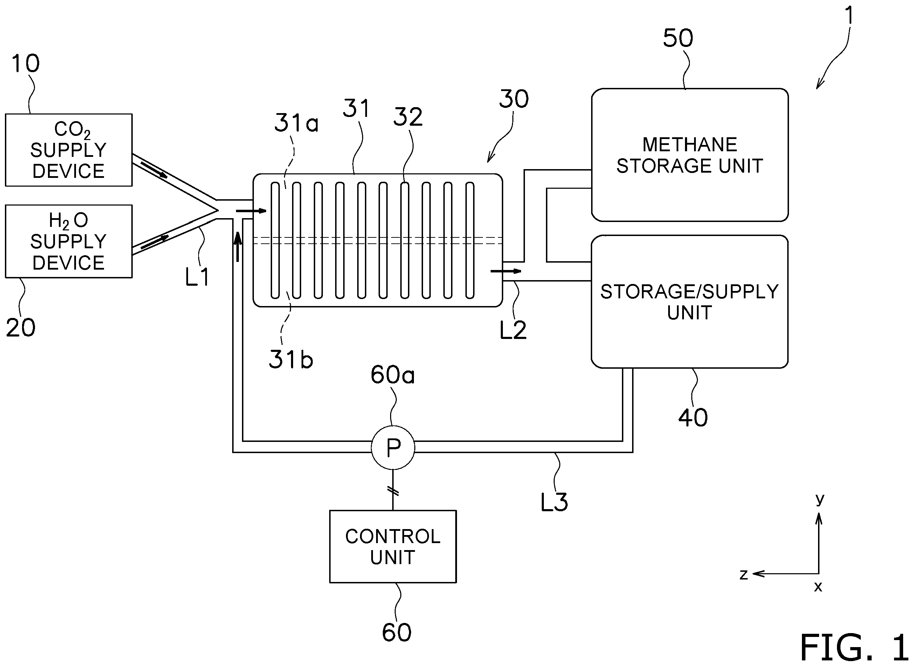

FIG. 1 is a block diagram showing a configuration of a methane production system 1 according to this embodiment.

The methane production system 1 includes a COz supply device 10, an $H_2O$ supply device 20, a co-electrolysis/reforming device 30, a storage/supply unit 40, a methane storage unit 50, and a control unit 60.

The COz supply device 10 is connected to the co-electrolysis/reforming device 30 via a first pipe L1. When each co-electrolysis/reforming cell 32, which will be described later, operates in a co-electrolysis mode, the COz supply device 10 supplies COz (carbon dioxide) to the co-electrolysis/reforming device 30. It is preferable that the amount of $CO_2$ supplied from the COz supply device 10 to the co-electrolysis/reforming device 30 is constant. Accordingly, it is possible to suppress the production of C (solid carbon) and COz due to disproportionation reaction of CO (carbon monoxide) produced in the co-electrolysis/reforming cells 32 of the co-electrolysis/reforming device 30. As a result, it is possible to suppress deterioration of the electrode activity of the first electrode 2 of each co-electrolysis/reforming cell 32, which will be described later. When each co-electrolysis/reforming cell 32, which will be described later, operates in a reforming mode, the COz supply device 10 does not supply COz to the co-electrolysis/reforming device 30.

The $H_2O$ supply device 20 is connected to the co-electrolysis/reforming device 30 via the first pipe L1. When each co-electrolysis/reforming cell 32 operates in the co-electrolysis mode, the $H_2O$ supply device 20 supplies $H_2O$ (water content) to the co-electrolysis/reforming device 30. The entirety or most of $H_2O$ supplied from the $H_2O$ supply device 20 to the co-electrolysis/reforming device 30 is gas (steam), but part of the $H_2O$ may be liquid (water). When each co-electrolysis/reforming cell 32 operates in the reforming mode, the $H_2O$ supply device 20 does not supply $H_2O$ to the co-electrolysis/reforming device 30.

The co-electrolysis/reforming device 30 includes a manifold 31 and a plurality of co-electrolysis/reforming cells 32.

The manifold 31 has a configuration in which gas can be distributed to the co-electrolysis/reforming cells 32 and gas can be collected from the co-electrolysis/reforming device. The manifold 31 internally has a gas supply chamber 31a and a gas collection chamber 31b. The gas supply chamber 31a and the gas collection chamber 31b are airtightly separated from each other.

The first pipe L1 is connected to the gas supply chamber 31a. In the co-electrolysis mode, COz and $H_2O$ are supplied from the first pipe L1 to the gas supply chamber 31a. In the reforming mode, $H_2$ and CO are supplied from the first pipe L1 to the gas supply chamber 31a.

The gas collection chamber 31b collects gas produced in the co-electrolysis/reforming cells 32. A second pipe L2 is connected to the gas collection chamber 31b. In the co-electrolysis mode, $H_2$ and CO are discharged from the gas collection chamber 3 1b to the second pipe L2. In the reforming mode, $CH_4$ and $H_2O$ are discharged from the gas collection chamber 31b to the second pipe L2. A reforming catalyst may be placed in the gas collection chamber 31b. The reforming catalyst may be in the form of pellets. The gas collection chamber 31b may be filled with the reforming catalyst. It is possible to use $Ru/Al_2O_3$, $Ni/Al_2O_3$, or the like as a reforming catalyst, for example.

A base end portion of each co-electrolysis/reforming cell 32 is supported by the manifold 31. A leading end portion of each co-electrolysis/reforming cell 32 is a free end. The number of co-electrolysis/reforming cells 32 is not particularly limited as long as it is 1 or more.

The co-electrolysis/reforming cells 32 operate in either the co-electrolysis mode in which $H_2$ (hydrogen), CO, Oz (oxygen) are produced from COz and HzO, or the reforming mode in which $CH_4$ (methane) is produced from the $H_2$ and CO produced in the co-electrolysis mode. The term "co-electrolysis" used in this specification refers to production of $H_2$, CO, and Oz by electrolyzing COz and $H_2O$ together. The term "reforming" used in this specification refers to production of $CH_4$ and $H_2O$ from $H_2$ and CO.

Each co-electrolysis/reforming cell 32 operates at high temperatures (e.g., 600°C to 850°C) in the co-electrolysis mode. The operating temperatures of the co-electrolysis/reforming cells 32 in the co-electrolysis mode are preferably 700°C or more and 850°C or less. By setting the operating temperature thereof to 700°C or more, the CO concentration can be increased due to thermodynamic equilibrium. By setting the operating temperature thereof to 850°C or less, the amount of oxide ions flowing through the interconnector can be suppressed.

In the reforming mode, each co-electrolysis/reforming cell 32 operates at a temperature (e.g., 200°C to 500°C) lower than the operating temperature of the co-electrolysis/reforming cell 32 in the co-electrolysis mode. The operating temperatures of the co-electrolysis/reforming cells 32 in the reforming mode are preferably 350°C or more and 400°C or less. By setting the operating temperature thereof to 350°C or more, it is possible to suppress the phase transformation of constituent materials of the support substrate 35 to carbonates. By setting the operating temperature thereof to 400°C or less, it is possible to inhibit the produced $CH_4$ from being re-reformed to $H_2$ and CO.

The wording "operating temperature of the co-electrolysis/reforming cell 32" used in this specification refers to the temperature at the center in the longitudinal direction (X-axis direction) of the co-electrolysis/reforming cell 32. The operating temperature of each co-electrolysis/reforming cell 32 is controlled by the control unit 60.

In the co-electrolysis mode, COz and $H_2O$ are supplied from the gas supply chamber 31a to the co-electrolysis/reforming cells 32. In the co-electrolysis/reforming cells 32, $H_2$, CO, and $O^{2-}$ (oxygen ions) are produced from COz and $H_2O$ at the first electrode 2, and Oz is produced from $O^{2-}$ at the second electrode 4. The produced $H_2$ and CO are collected into the gas collection chamber 31b and discharged from the second pipe L2.

In the reforming mode, $H_2$ and CO are supplied from the gas supply chamber 31a to the co-electrolysis/reforming cells 32. In the co-electrolysis/reforming cells 32, $CH_4$ and $H_2O$ are produced from $H_2$ and CO at the first electrode 2. The produced $CH_4$ and $H_2O$ are collected into the gas collection chamber 31b and discharged from the second pipe L2.

An example of a configuration of the co-electrolysis/reforming device 30 will be described later.

The storage/supply unit 40 is connected to the co-electrolysis/reforming device 30. In this embodiment, the storage/supply unit 40 is connected to the co-electrolysis/reforming device 30 via the second pipe L2 and the third pipe L3. Specifically, the storage/supply unit 40 is directly connected to the gas collection chamber 31b of the co-electrolysis/reforming device 30 by the second pipe L2, and directly connected to the gas supply chamber 31a of the co-electrolysis/reforming device 30 by the third pipe L3.

When the co-electrolysis/reforming cells 32 operate in the co-electrolysis mode, the storage/supply unit 40 stores $H_2$ and CO discharged from the gas collection chamber 31b via the second pipe L2. Therefore, the $H_2$ and CO produced in the co-electrolysis/reforming device 30 are directly stored in the storage/supply unit 40 without changing their compositions.

When the co-electrolysis/reforming cells 32 operate in the reforming mode, the storage/supply unit 40 supplies the stored $H_2$ and CO to the gas supply chamber 31a via the third pipe L3. Therefore, the $H_2$ and CO produced in the co-electrolysis/reforming device 30 are directly returned to the co-electrolysis/reforming device 30 without changing their compositions. When the co-electrolysis/reforming cells 32 operate in the reforming mode, the storage/supply unit 40 does not receive gas discharged from the co-electrolysis/reforming device 30.

The methane storage unit 50 is connected to the co-electrolysis/reforming device 30. In this embodiment, the methane storage unit 50 is connected to the co-electrolysis/reforming device 30 via the second pipe L2. Specifically, the methane storage unit 50 is directly connected to the gas collection chamber 31b of the co-electrolysis/reforming device 30 by the second pipe L2.

When the co-electrolysis/reforming cells 32 operate in the reforming mode, the methane storage unit 50 stores $CH_4$ and $H_2O$ discharged from the gas collection chamber 31b via the second pipe L2. When the co-electrolysis/reforming cells 32 operate in the co-electrolysis mode, the methane storage unit 50 does not receive gas discharged from the co-electrolysis/reforming device 30.

The control unit 60 controls the operating temperature of each co-electrolysis/reforming cell 32. The control unit 60 can control the operating temperature of the co-electrolysis/reforming cell 32 by adjusting, for example, at least one of a current value, the amount of gas supplied to the first electrode 2, and the amount of gas supplied to the first electrode 2, which will be described later. In this embodiment, the control unit 60 makes the operating temperature of the co-electrolysis/reforming cell 32 in the reforming mode lower than the operating temperature of the co-electrolysis/reforming cell 32 in the co-electrolysis mode.

When the co-electrolysis/reforming cells 32 operate in the reforming mode, the control unit 60 drives a pump 60a arranged in the third pipe L3. As a result, the $H_2$ and CO stored in the storage/supply unit 40 are supplied to the gas supply chamber 31a of the co-electrolysis/reforming device 30 via the third pipe L3. When the co-electrolysis/reforming cells 32 operate in the co-electrolysis mode, the control unit 60 does not drive the pump 60a.

Configuration of co-electrolysis/reforming device 30

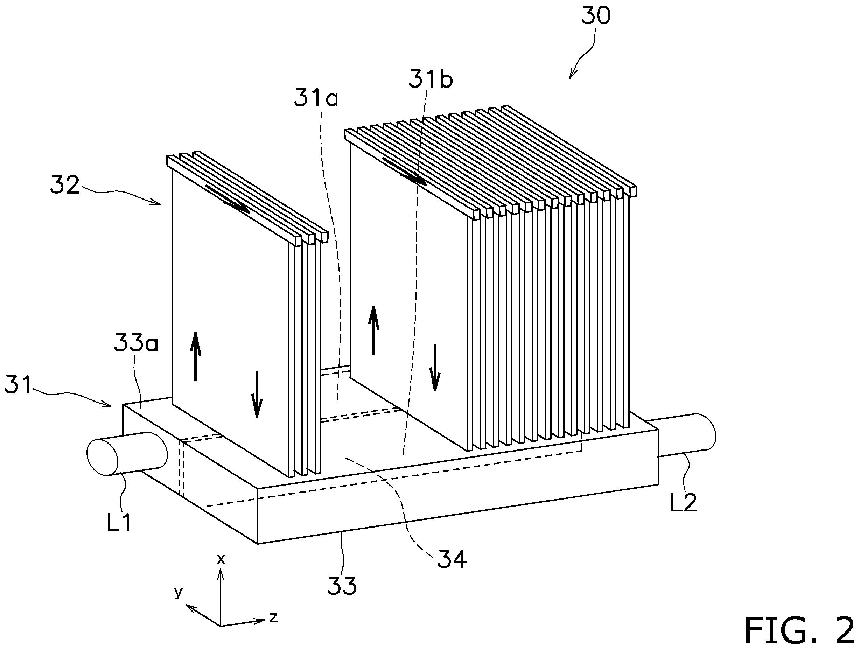

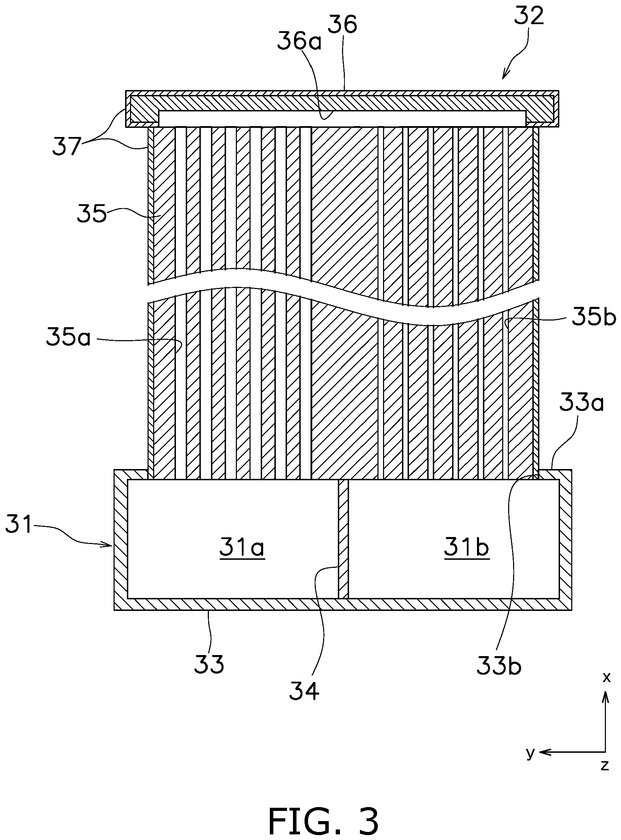

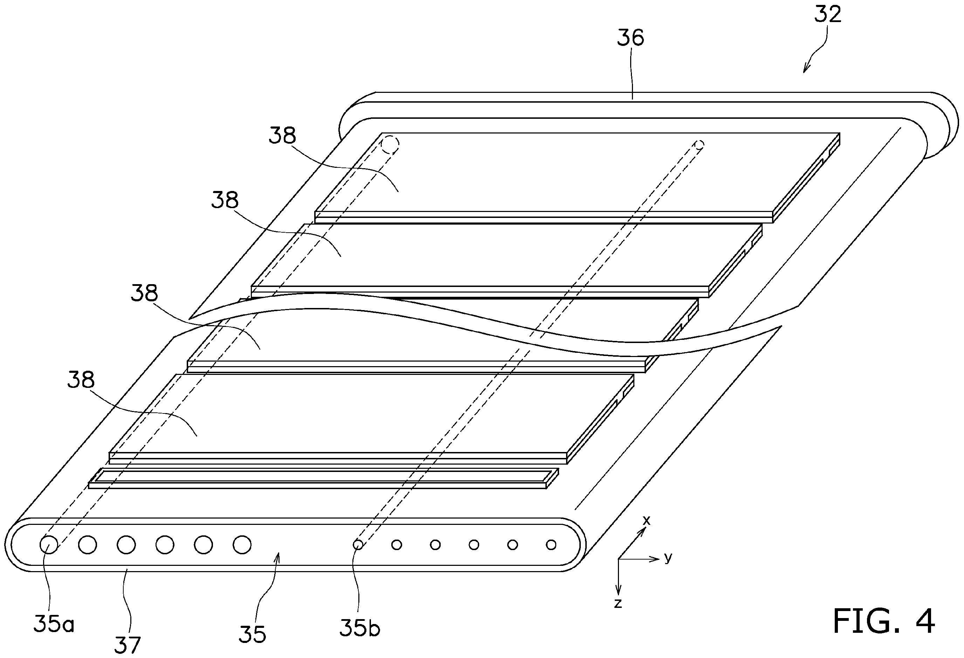

FIG. 2 is a perspective view of the co-electrolysis/reforming device 30. FIG. 3 is a cross-sectional view of the co-electrolysis/reforming device 30. FIG. 4 is a perspective view of the co-electrolysis/reforming device 32. Some of the co-electrolysis/reforming cells 32 are not shown in FIG. 2.

Manifold 31

As shown in FIGS. 2 and 3, the manifold 31 includes a manifold main body portion 33 and a partition plate 34.

The manifold main body portion 33 is hollow. The partition plate 34 is arranged in the manifold main body portion 33. The partition plate 34 airtightly separates the gas supply chamber 31a and the gas collection chamber 31b from each other.

The manifold main body portion 33 has a top plate portion 33a. As shown in FIG. 3, the top plate portion 33a is provided with a plurality of through holes 33b. The through holes 33b are arranged side-by-side at predetermined intervals in the longitudinal direction (Z-axis direction) of the manifold main body portion 33. Each through hole 33b extends in the width direction (Y-axis direction) of the manifold main body portion 33. Although each through hole 33b is a long hole that is in communication with the gas supply chamber 31a and the gas collection chamber 31b in this embodiment, the through hole 33b may be divided into a hole that is in communication with the gas supply chamber 31a and a hole that is in communication with the gas collection chamber 31b.

Co-electrolysis/reforming cell 32

As shown in FIGS. 2 and 3, each co-electrolysis/reforming cell 32 extends in a direction away from the manifold 31. A base end portion of each co-electrolysis/reforming cell 32 is fixed to the through hole 33b of the top plate portion 33a using a bonding material (not shown) or the like. The base end portion of the co-electrolysis/reforming cell 32 may be inserted into the through hole 33b, or may protrude outward of the through hole 33b.

The co-electrolysis/reforming cells 32 are disposed such that their main surfaces face each other. The co-electrolysis/reforming cells 32 are arranged side-by-side at predetermined intervals along the longitudinal direction (Z-axis direction) of the manifold 31. That is, the arrangement direction of the co-electrolysis/reforming cells 32 extends in the longitudinal direction of the manifold 31. The co-electrolysis/reforming cells 32 are electrically connected in series or in a combination of series and parallel connections, using current collector members (not shown).

As shown in FIGS. 3 and 4, each co-electrolysis/reforming cell 32 includes a support substrate 35, a connection member 36, a coating layer 37, and a plurality of element portions 38. The co-electrolysis/reforming cell 32 according to this embodiment is a so-called horizontal-stripe type solid oxide electrolysis cell (SOEC).

The support substrate 35 is plate-shaped. In this embodiment, the vertical direction (X-axis direction) in FIG. 3 is the longitudinal direction of the support substrate 35, and the horizontal direction (Y-axis direction) in FIG. 3 is the width direction of the support substrate 35.

A plurality of first gas channels 35a and a plurality of second gas channels 35b are formed in the support substrate 35. The first gas channels 35a and the second gas channels 35b each extend from the base end portion to the leading end portion of the co-electrolysis/reforming cell 32 in the support substrate 35. The first gas channels 35a and the second gas channels 35b pass through the support substrate 35. The first gas channels 35a are disposed at intervals in the width direction of the support substrate 35. The second gas channels 35b are disposed at intervals in the width direction of the support substrate 35. Although the inner diameter of the first gas channels 35a is larger than the inner diameter of the second gas channels 35b in this embodiment, the inner diameter of the first gas channels 35a and the inner diameter of the second gas channels 35b are not particularly limited.

The first gas channels 35a are open to the gas supply chamber 31a. Gas flows into the first gas channels 35a from the gas supply chamber 31a. The second gas channels 35b are open to the gas collection chamber 31b. Gas flows out from the second gas channels 35b and enters the gas collection chamber 31b.

The support substrate 35 is made of a porous material having no electron conductivity so as to allow gas permeation while preventing short circuits between element portions 38. The support substrate 35 may be made of CSZ (calcia-stabilized zirconia), 8YSZ (yttria-stabilized zirconia), $Y_2O_3$ (yttria), MgO (magnesium oxide), $MgAl_2O_4$ (magnesia alumina spinel), or a composite thereof, for example. The support substrate 35 may have a porosity of 20% to 60%. Note that the porosity mentioned in this specification is a value measured using the Archimedes' method.

The connection member 36 is attached to the leading end portion of the support substrate 35. The connection member 36 may be made of a porous material similar to that of the support substrate 35, for example. The connection member 36 internally has a connection channel 36a. The connection channel 36a is in communication with the first gas channels 35a and the second gas channels 35b.

The coating layer 37 covers outer surfaces of the support substrate 35 and the connection member 36. The coating layer 37 is denser than the support substrate 35 and the connection member 36. The coating layer 37 may have a porosity of about 0% to 7%. The coating layer 37 may be made of a material used in the later-described electrolyte 3, crystallized glass, or the like.

The element portions 38 are supported by the support substrate 35. The element portions 38 may be arranged on both main surfaces of the support substrate 35, or may be arranged on only one of the main surfaces.

Element portion 38

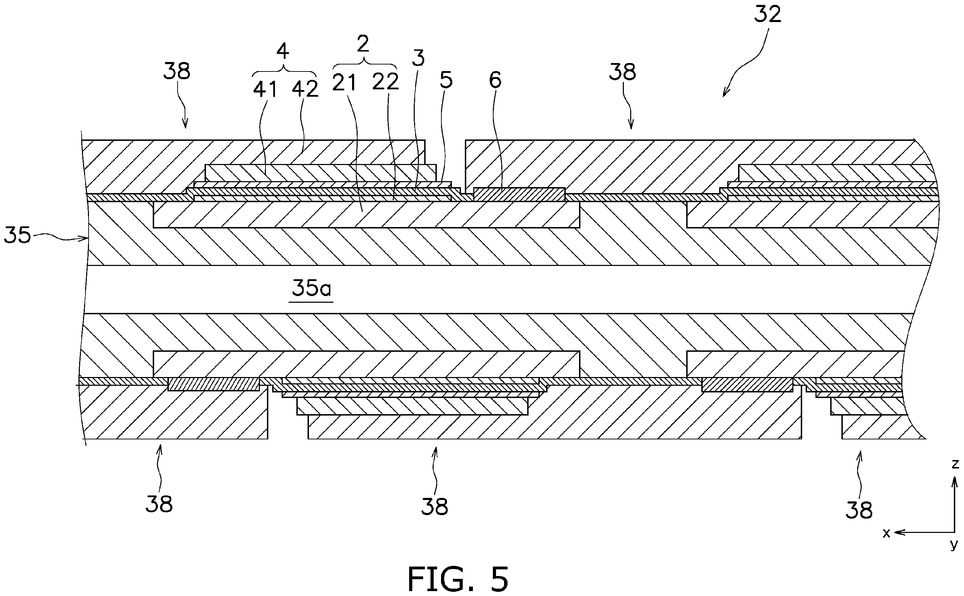

FIG. 5 is a cross-sectional view of the co-electrolysis/reforming cell 32 cut along the first gas channel 35a.

Each element portion 38 has a first electrode 2, an electrolyte 3, a second electrode 4, a reaction preventing film 5, and an interconnector 6.

When the co-electrolysis/reforming cell 32 operates in the co-electrolysis mode, at the first electrode 2, $H_2$, CO, and $O^{2-}$ are produced from COz and $H_2O$ according to the chemical reaction of the co-electrolysis indicated by Chemical Equation (1) below.

- First electrode 2 (co-electrolysis mode):

$$CO_2 + H_2O + 4e^- \rightarrow CO + H_2 + 2O^{2-} \qquad (1)$$

When the co-electrolysis/reforming cell 32 operates in the reforming mode, at the first electrode 2, $CH_4$ and $H_2O$ are produced from $H_2$ and CO according to the chemical reaction of the reforming indicated by Chemical Equation (2) below.

- First electrode 2 (reforming mode):

$$3H_2 + CO \rightarrow CH_4 + H_2O \qquad (2)$$

The first electrode 2 has a first electrode base body 21 and a first electrode active portion 22.

The first electrode base body 21 is disposed on the support substrate 35. The first electrode base body 21 is embedded in a recess formed in a surface of the support substrate 35 in this embodiment, but may be placed on the surface of the support substrate 35. The first electrode base body 21 may have a thickness of 50 to 500 $\mu$m.

The first electrode base body 21 is made of a porous material having electron conductivity. The first electrode base body 21 preferably has higher electron conductivity than the first electrode active portion 22. The first electrode base body 21 optionally has oxygen ion conductivity. The first electrode base body 21 may be made of a composite of NiO and 8YSZ, a composite of NiO and $Y_2O_3$, a composite of NiO and CSZ, or the like, for example.

The first electrode active portion 22 is disposed on the first electrode base body 21. The first electrode active portion 22 may have a thickness of 5 to 100 $\mu$m. The first electrode active portion 22 has oxygen ion conductivity and electron conductivity. The first electrode active portion 22 preferably has higher oxygen ion conductivity than the first electrode base body 21. The first electrode active portion 22 may be made of a composite of NiO and 8YSZ, a composite of NiO and GDC (Ce, Gd)Oz (gadolinium doped ceria), or the like, for example.

The electrolyte 3 is disposed between the first electrode 2 and the second electrode 4. The electrolyte 3 transfers $O^{2-}$ produced at the first electrode 2 to the second electrode 4. The electrolyte 3 is disposed on the first electrode 2. In this embodiment, the electrolyte 3 extends in the longitudinal direction of the support substrate 35 between two interconnectors 6. The electrolyte 3 may have a thickness of 3 to 50 $\mu$m, for example.

The electrolyte 3 is made of a dense material that has oxygen ion conductivity and does not have electron conductivity. The electrolyte 3 is denser than the support substrate 35. The electrolyte 3 may have a porosity of 0% to 7%, for example. The electrolyte 3 may be made of 8YSZ, LSGM (lanthanum gallate), or the like, for example.

When the co-electrolysis/reforming cell 32 operates in the co-electrolysis mode, at the second electrode 4, Oz is produced from $O^{2-}$ transferred from the first electrode 2 through the electrolyte 3, according to the chemical reaction indicated by Chemical Equation (3) below.

- 

$$\text{Second electrode 4 (co-electrolysis mode): } 2O^{2-} \rightarrow O_2 + 4e^- \cdots (3)$$

When the co-electrolysis/reforming cell 32 operates in the reforming mode, the second electrode 4 is not particularly functional.

The second electrode 4 has a second electrode active portion 41 and a second electrode base body 42.

The second electrode active portion 41 is disposed on the reaction preventing film 5. The second electrode active portion 41 may have a thickness of 10 to 100 $\mu$m, for example.

The second electrode active portion 41 is made of a porous material having oxygen ion conductivity and electron conductivity. The second electrode active portion 41 preferably has higher oxygen ion conductivity than the second electrode base body 42. The second electrode active portion 41 may be made of LSCF=(La, Sr)(Co, Fe)Os (lanthanum strontium cobalt ferrite), LSF=(La, Sr) $FeO_3$ (lanthanum strontium ferrite), LNF=La(Ni, Fe)$O_3$ (lanthanum nickel ferrite), LSC=(La, Sr)$CoO_3$ (lanthanum strontium cobaltite), SSC=(Sm, Sr)CoOs (samarium strontium cobaltite), or the like, for example.

The second electrode base body 42 is disposed on the second electrode active portion 41. The second electrode base body 42 is electrically connected to the first electrode base body 21 of the adjacent element portion 38 via the interconnector 6. The second electrode base body 42 may have a thickness of 50 to 500 $\mu$m, for example.

The second electrode base body 42 is made of a porous material having electron conductivity. The second electrode base body 42 preferably has higher electron conductivity than the second electrode active portion 41. The second electrode base body 42 optionally has oxygen ion conductivity. The second electrode base body 42 may be made of LSCF, LSC, Ag (silver), Ag-Pd (silver palladium alloy), or the like, for example.

The reaction preventing film 5 is disposed between the electrolyte 3 and the second electrode active portion 41. The reaction preventing film 5 suppresses a reaction of substances contained in the electrolyte 3 and the second electrode active portion 41 to form a reaction layer having high electric resistance. The reaction preventing film 5 may have a thickness of 3 to 50 $\mu$m, for example. The reaction preventing film 5 is made of a dense material. The reaction preventing film 5 may be made of GDC, for example.

The interconnector 6 is connected to the second electrode base body 42 and the first electrode base body 21 of the adjacent element portion 38. The interconnector 6 may have a thickness of 10 to 100 $\mu$m, for example. The interconnector 6 is made of a dense material that have electron conductivity. The interconnector 6 is denser than the support substrate 35. The interconnector 6 may have a porosity of 0% to 7%. The interconnector 6 may be made of $LaCrO_3$ (lanthanum chromite), (Sr, La)$TiO_3$ (strontium titanate), or the like, for example.

Methane production method

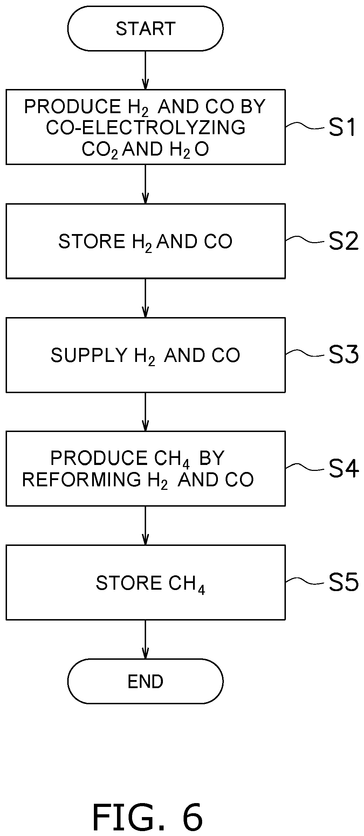

FIG. 6 is a flowchart illustrating a methane production method using the co-electrolysis/reforming cells 32.

In step S1, the co-electrolysis/reforming cells 32 produce $H_2$ and CO at the first electrode 2 by co-electrolyzing $CO_2$ and $H_2O$ (co-electrolyzing step).

In step S2, the storage/supply unit 40 stores the $H_2$ and CO produced in the co-electrolysis/reforming cells 32 (first storing step).

In step S3, the storage/supply unit 40 supplies the stored $H_2$ and CO to the co-electrolysis/reforming cells 32 (supplying step).

In step S4, the co-electrolysis/reforming cells 32 produce $CH_4$ by reforming $H_2$ and CO (reforming step).

In step S5, the methane storage unit 50 stores the $CH_4$ produced in the co-electrolysis/reforming cells 32 (second storing step).

Features

The methane production system 1 includes the co-electrolysis/reforming cells 32 and the control unit 60 that controls the operating temperatures of the co-electrolysis/reforming cells 32. The co-electrolysis/reforming cells 32 operate in either the co-electrolysis mode in which $H_2$ and CO are produced at the first electrode 2 from $CO_2$ and $H_2O$, or the reforming mode in which $CH_4$ is produced at the first electrode 2 from the $H_2$ and CO produced in the co-electrolysis mode. The control unit 60 makes the operating temperatures of the co-electrolysis/reforming cells 32 in the reforming mode lower than the operating temperatures of the co-electrolysis/reforming cells 32 in the co-electrolysis mode.

It is possible to produce $CH_4$ in the co-electrolysis/reforming cells 32 on-site, using the $H_2$ and CO produced in the co-electrolysis/reforming cells 32. Therefore, $H_2$ and CO do not need to be transported from a plant where a co-electrolysis device is installed to a plant where a reforming device is installed.

Variation of embodiment

Although an embodiment of the present invention has been described above, the present invention is not limited thereto, and various modifications can be made without departing from the spirit of the present invention.

Variation 1

Although the co-electrolysis/reforming cell 32 is a horizontal-stripe type SOEC in the above embodiment, the co-electrolysis/reforming cell 32 is not limited to this. The co-electrolysis/reforming cell 32 may be a vertical-stripe type (hollow flat plate type), flat plate type, or cylindrical type SOEC, or the like. A configuration of a vertical-stripe type SOEC is described in JP 2015-125897A, for example. A configuration of a flat plate type SOEC is described in JP 2020-177839A, for example. A configuration of a cylindrical type SOEC is described in JP 2008-270203A, for example. However, horizontal-stripe type SOECs are particularly preferable because they have higher $H_2O$ utilization efficiency than other SOECs.

Variation 2

Although each element portion 38 has the first electrode 2, the electrolyte 3, the second electrode 4, the reaction preventing film 5, and the interconnector 6 in the above embodiment, it is sufficient that the element portion 38 includes at least the first electrode 2, the electrolyte 3, and the second electrode 4.

Variation 3

When the co-electrolysis/reforming cells 32 operate in the reforming mode, the control unit 60 drives the pump 60a arranged in the third pipe L3 so as to supply $H_2$ and CO from the storage/supply unit 40 to the co-electrolysis/reforming device 30 in the above embodiment. However, the present invention is not limited to this. If pressure is applied to the $H_2$ and CO stored in the storage/supply unit 40, for example, the control unit 60 may adjust the opening degree of a flow control valve provided instead of the pump 60a so as to supply $H_2$ and CO from the storage/supply unit 40 to the co-electrolysis/reforming device 30.

REFERENCE SIGNS LIST

| | |
|---|---|
| 1 | Methane production system |
| 10 | $CO_2$ supply device |
| 20 | $H_2O$ supply device |
| 30 | Co-electrolysis/reforming device |
| 31 | Manifold |
| 32 | Co-electrolysis/reforming cell |
| 38 | Element portion |
| 2 | First electrode |
| 3 | Electrolyte |
| 4 | Second electrode |
| 40 | Storage/supply unit |
| 50 | Methane storage unit |
| 60 | Control unit |
| L1 | First pipe |
| L2 | Second pipe |
| L3 | Third pipe |

**Claims**

1.  A methane production system comprising:

    a co-electrolysis/reforming cell having a first electrode, a second electrode, and an electrolyte disposed between the first electrode and the second electrode; and
    a control unit configured to control an operating temperature of the co-electrolysis/reforming cell, wherein
    the co-electrolysis/reforming cell operates in either a co-electrolysis mode in which $H_2$ and CO are produced at the first electrode from $CO_2$ and $H_2O$, or a reforming mode in which $CH_4$ is produced at the first electrode from the $H_2$ and CO produced in the co-electrolysis mode, and
    the control unit makes an operating temperature of the co-electrolysis/reforming cell in the reforming mode lower than an operating temperature of the co-electrolysis/reforming cell in the co-electrolysis mode.

2.  The methane production system according to claim 1, wherein

    the operating temperature of the co-electrolysis/reforming cell in the co-electrolysis mode is 700°C or more and 850°C or less, and
    the operating temperature of the co-electrolysis/reforming cell in the reforming mode is 350°C or more and 400°C or less.

3.  The methane production system according to claim 1 or 2, further comprising
    a storage/supply unit configured to store the $H_2$ and CO produced at the first electrode when the co-electrolysis/reforming cell operates in the co-electrolysis mode, and supply the stored $H_2$ and CO to the first electrode when the co-electrolysis/reforming cell operates in the reforming mode.

4. A methane production method using a co-electrolysis/reforming cell having a first electrode, a second electrode, and an electrolyte disposed between the first electrode and the second electrode, the method comprising:

a co-electrolyzing step of producing $H_2$ and CO at the first electrode from $CO_2$ and $H_2O$; and
a reforming step of producing $CH_4$ at the first electrode from the $H_2$ and CO produced in the co-electrolyzing step.

5. The methane production method according to claim 4, further comprising

a first storing step of storing the $H_2$ and CO produced in the co-electrolyzing step, wherein,
in the reforming step, $CH_4$ is produced from the $H_2$ and CO stored in the storing step.

6. The methane production method according to claim 5, further comprising a second storing step of storing the $CH_4$ produced in the reforming step.

FIG. 1

FIG. 2

FIG. 3

FIG. 4

FIG. 5

START

PRODUCE $H_2$ AND CO BY CO-ELECTROLYZING $CO_2$ AND $H_2O$ — S1

STORE $H_2$ AND CO — S2

SUPPLY $H_2$ AND CO — S3

PRODUCE $CH_4$ BY REFORMING $H_2$ AND CO — S4

STORE $CH_4$ — S5

END

# FIG. 6

## INTERNATIONAL SEARCH REPORT

| International application No. |
|---|
| **PCT/JP2022/009406** |

**A. CLASSIFICATION OF SUBJECT MATTER**

*C07C 1/04*(2006.01)i; *C07C 9/04*(2006.01)i; *C25B 1/23*(2021.01)i
FI:  C07C1/04; C07C9/04; C25B1/23

According to International Patent Classification (IPC) or to both national classification and IPC

**B. FIELDS SEARCHED**

Minimum documentation searched (classification system followed by classification symbols)

C07C1/04; C07C9/04; C25B1/23

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2019-112717 A (COMMISSARIAT A L'ENERGIE ATOMIQUE ET AUX ENERGIES ALTERNATIVES) 11 July 2019 (2019-07-11)<br>claims, paragraphs [0040], [0046]-[0047], [0076]-[0096], fig. 3 | 1-6 |
| X | WO 2020/071376 A1 (NATIONAL INSTITUTE OF ADVANCED INDUSTRIAL SCIENCE AND TECHNOLOGY) 09 April 2020 (2020-04-09)<br>claims, example 2, fig. 2 | 1-6 |
| A | CHEN, Long et al. Direct synthesis of methane from CO2-H2O co-electrolysis in tubular solid oxide electrolysis cells. Energy and Environmental Science. 2014, vol. 7, pp. 4018-4022, ISSN 1754-5692<br>fig. 1 | 1-6 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **17 May 2022** | **24 May 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

<table>
<tr><th colspan="2">INTERNATIONAL SEARCH REPORT<br>Information on patent family members</th><th colspan="2">International application No.<br><br>**PCT/JP2022/009406**</th></tr>
<tr><th>Patent document<br>cited in search report</th><th>Publication date<br>(day/month/year)</th><th>Patent family member(s)</th><th>Publication date<br>(day/month/year)</th></tr>
<tr><td>JP 2019-112717 A</td><td>11 July 2019</td><td>US 2019/0194816 A1<br>claims, paragraphs [0044],<br>[0050]-[0051], [0089]-[0122],<br>fig. 3<br>EP 3502317 A1<br>FR 3075832 A<br>CA 3028495 A1</td><td></td></tr>
<tr><td>WO 2020/071376 A1</td><td>09 April 2020</td><td>CN 112770837 A</td><td></td></tr>
</table>

Form PCT/ISA/210 (patent family annex) (January 2015)

**EP 4 306 502 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018154864 A
- JP 2019175636 A
- JP 2015125897 A
- JP 2020177839 A
- JP 2008270203 A